# EUROPÄISCHE PATENTANMELDUNG

(11) **EP 0 676 184 A1**
(43) Veröffentlichungstag der Anmeldung: **11.10.1995**
(21) Anmeldenummer: 95101965.2
(22) Anmeldetag: 14.02.1995
(51) Int. Cl.: A61F 13/66, A41B 13/04

(54) **Windelhose**

(30) Priorität: 22.02.1994 DE 4405521
(71) Anmelder: Gräther, Dieter, D-72818 Trochtelfingen (DE)
(72) Erfinder: Gräther, Dieter, D-72818 Trochtelfingen (DE)
(74) Vertreter: Möbus, Rudolf, Dipl.-Ing.

(57) **Zusammenfassung**

Eine Windelhose (10) zum Einlegen einer Windel mit einem hinteren Bundteil (11), einem Mittelteil (12) und einem vorderen Bundteil (13) ist so gestaltet, daß auf den Seiten der Windelhose flexible Pufferzonen vorgesehen sind, die aus einer Pufferzone (18.1, 18.2) bestehen, in der sich ein Puffergummiband (20.1, 20.2) befindet. Die Pufferzonen ermöglichen ein flexibles Ändern des Umfangs im Bundbereich und verhindern dadurch ein Verrutschen der Windel beim Tragen.

## Beschreibung

Die Erfindung betrifft eine Windelhose zum Einlegen einer Windel mit einem hinteren Bundteil, einem Mittelteil und einem vorderen Bundteil, bei der der hintere Bundteil Verschlußlaschen und einen Stofftunnel aufweist, in der ein Bundgummiband geführt ist, das auf beiden Längsenden des Stofftunnels an dem Stofftunnel fixiert ist und bei der der vordere Bundteil eine Auflagefläche zur Befestigung der Verschlußlaschen hat.

Derartige Windelhosen werden dazu verwendet, bei Säuglingen und Kleinkindern sowie inkontinenten Erwachsenen eine Windel im Beckenbereich fest am Körper anliegend zu halten. Die gattungsgemäße Windelhose wird so getragen, daß der hintere Bundteil am Rücken und der vordere Bundteil am Bauch anliegt, während der Mittelteil zwischen den Beinen eine eingelegte Windel trägt. Mit den zwei Verschlußlaschen wird der hintere Bundteil am vorderen Bundteil so befestigt, daß eine Hose entsteht.

Das Gummiband im hinteren Bundteil bringt den Vorteil, daß die Windelhose sehr stramm anliegend oder lockerer anliegend getragen werden kann. Dazu wird beim Anlegen der Windelhose das Gummiband mehr oder weniger stark gespannt. Es hat sich jedoch gezeigt, daß nach dem Anlegen der Windelhose das Gummiband im hinteren Bundteil Umfangsänderungen kaum noch ausgleichen kann. Insbesondere beim Sport oder beim Spielen der Kleinkinder werden sehr hohe Anforderungen an die Flexibilität der Windelhose im Bundbereich gestellt. Herkömmliche Windelhosen werden diesen Anforderungen nicht gerecht. Sie fangen an zu verrutschen, wenn die Windelträger Sport treiben oder wenn Kleinkinder wild herumtollen.

Der Erfindung liegt daher die Aufgabe zugrunde, eine Windelhose der eingangs genannten Art so auszubilden, daß sie im angelegten Zustand flexibel auf Umfangsänderungen im Bundbereich reagieren kann.

Die Aufgabe wird mit einer Windel der eingangs genannten Art erfindungsgemäß dadurch gelöst, daß zwischen dem Stofftunnel und mindestens einer Verschlußlasche eine Pufferzone angeordnet ist, in der ein Puffergummiband vorgesehen ist.

Wenn die Windel angelegt ist, liegt diese Pufferzone seitlich im vorderen Bereich der Windelhose. Vorteilhaft ist es, auf jeder Seite der Windelhose zwischen Stofftunnel und Verschlußlasche jeweils eine Pufferzone vorzusehen. Die Pufferzone mit dem darin angeordneten Puffergummiband bildet eine flexible Pufferzone, die besonders leicht auf Umfangsänderungen am Bund der Windelhose reagiert. Wenn beim Tragen der Windelhose im seitlichen Bereich Spannungen an der Hose auftreten, so werden diese von der flexiblen Pufferzone direkt abgefedert.

Vor allem werden auch Ausdehnungen im Bauchbereich, die bei Spiel und Sport auftreten oder nach einem ausgiebigen Essen, von der flexiblen Pufferzone ausgeglichen, ohne daß die Windel im Bundbereich geöffnet werden muß, um sie auf einen anderen Umfang einzustellen.

Vorteilhaft ist es, die Verschlußlaschen der Windelhose mit Klettbändern zu versehen und an den Auflageflächen Flauschflächen vorzusehen, die ein einfaches schnelles Anlegen der Windel ermöglichen.

Prinzipiell ist es möglich, den Stofftunnel im hinteren Bundteil und die Pufferzone aus einem flexiblen Stoffmaterial herzustellen. Es hat sich jedoch als günstig erwiesen, die gesamte Windelhose im wesentlichen aus Baumwolle zu fertigen und den Stofftunnel im hinteren Bundteil so zu raffen, daß sie auf eine Ausdehnung des Gummibandes reagieren kann. Ebenso kann der Stoff der Pufferzone stark gerafft sein, um leicht und flexibel Ausdehnungen des Puffergummibandes zuzulassen.

Bundgummiband und Puffergummiband können auch aus einem einzigen durchgehenden Gummiband bestehen. Dies ist fertigungstechnisch besonders günstig, da bei der Fertigung ein Gummi-band in einen durchgehenden Stofftunnel im hinteren Bundteil eingelegt werden kann. Dieser durchgehende Stofftunnel wird durch Nähte quer zur Gummibandrichtung so unterteilt, daß ein Stofftunnel in der Mitte des hinteren Bundteils entsteht und zwei Pufferzonen an den Seiten des hinteren Bundteils.

Vorteilhaft ist es auch, im hinteren und im vorderen Bundteil Klettbänder vorzusehen, an die über Flauschflächen eine Windel befestigt werden kann. Diese Klettbänder können direkt unterhalb des Bundes der Windelhose verdeckt angeordnet werden, so daß die Klettfläche beim Tragen der Windelhose vom Körper abgewendet ist. Den Klettbändern gegenüber kann ebenfalls eine Flauschfläche angeordnet sein, die bei eingelegter Windel keine Funktion hat, aber beim Waschen der Windelhose dazu dient, das Klettband an der Windelhose zu halten.

Neuartige Materialien erlauben es, die Windelhose aus einem wasserundurchlässigen aber atmungsaktiven Material herzustellen. Dazu kann das Baumwollmaterial auf der Innenseite der Windelhose mit einer speziellen Oberfläche versehen sein. Derartige Beschichtungen gestatten es, die Windelhose bei 60° zu waschen und garantieren gleichzeitig die Kompostierbarkeit des Materials.

Zusammen mit einer in dem Gebrauchsmuster G 94 01 412 beschriebenen Windel, die ebenfalls an den Seiten des Mittelteils ein außen gerafftes Ansatzstück als Auslaufschutz aufweisen kann, bildet diese Windelhose ein optimales System für inkontinente Personen und Wickelkinder.

Nachfolgend wird ein bevorzugtes Ausführungsbeispiel einer erfindungsgemäßen Windelhose anhand der Zeichnung näher erläutert.

Im einzelnen zeigen:
- Fig. 1: eine Draufsicht auf eine Windelhose;
- Fig. 2: einen zentralen Querschnitt durch die Windelhose nach Fig. 1 längs der Linie II-II.

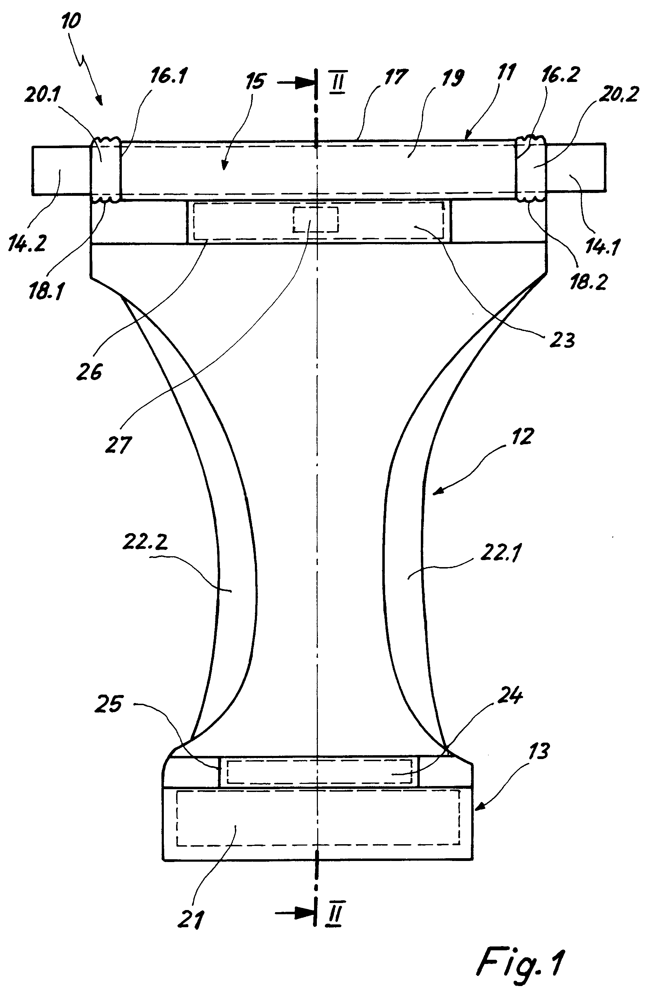

In Fig. 1 ist eine Windelhose 10 dargestellt, die im wesentlichen aus einem hinteren Bundteil 11, einem Mittelteil 12 und einem vorderen Bundteil 13 besteht. Der hintere Bundteil 11 hat an seinen beiden seitlichen Enden jeweils eine Verschlußlasche 14.1, 14.2, die aus einem Stück Klettband besteht, das an die restliche Windelhose angenäht ist. Der dazwischen liegende Bereich des hinteren Bundteils ist doppellagig ausgeführt und enthält ein Gummiband 15, das sich zwischen den Verschlußlaschen 14 erstreckt. Zwei Abnäher 16.1, 16.2 quer zur Laufrichtung des Gummibandes teilen das hintere Bundteil 11 in einen Stofftunnel 17 und zwei Pufferzonen 18.1, 18.2. Das durchgehende Gummiband 15 wird dadurch in ein Bundgummiband 19 und zwei Puffergummibänder 20.1, 20.2 unterteilt.

Der vordere Bundteil 13 hat eine Auflagefläche 21 für die Verschlußlaschen 14.1, 14.2, die aus einem Flauschband besteht, das außen auf die Windelhose im Bundbereich aufgenäht ist.

Das Mittelteil 12 ist mit einem sogenannten Auslaufschutz versehen. Dazu sind auf beiden Seiten des Mittelteils 12 außen geraffte Ansatzstücke 22.1, 22.2 vorgesehen, die sich beim Tragen der Windelhose eng an die Beine anlegen und somit ein Austreten von Feuchtigkeit aus der Windel im Beinbereich verhindern.

Sowohl am hinteren als auch am vorderen Bundteil 11, 13 sind Klettbänder 23, 24 direkt unterhalb des Bundteils vorgesehen. Diese Klettbänder dienen dazu, eine Windel festzuhalten, die an den entsprechenden Stellen mit Flauschflächen versehen ist (nicht gezeigt). Die Klettbänder 23, 24 sind jeweils unter einem Stoffband 25, 26 verdeckt angeordnet, so daß sie beim Tragen der Windelhose 10 nicht mit dem Körper in Berührung kommen. Zum Einlegen der Windel wird das Stoffband 25 bzw. 26 leicht angehoben, um die Windel unter das Stoffband zu schieben und dort an den Klettbändern 23, 24 zu befestigen. Den Klettbändern 23, 24 gegenüberliegend kann eine Flauschfläche 27 angeordnet sein, die vor allem beim Waschen der Windelhose das Klettband an der Windelhose hält.

Die gesamte Windelhose ist mit Ausnahme der Klettbänder und der Flauschflächen aus Baumwolle gefertigt. Der verwendete Baumwollstoff ist wasserundurchlässig aber atmungsaktiv beschichtet und im Bundbereich so umgeknickt, daß im Bereich des Mittelteils 12 die beschichtete Seite des Stoffs zur eingelegten Windel zeigt, im Bundbereich aber die unbeschichtete Seite des Baumwollstoffs am Körper anliegt.

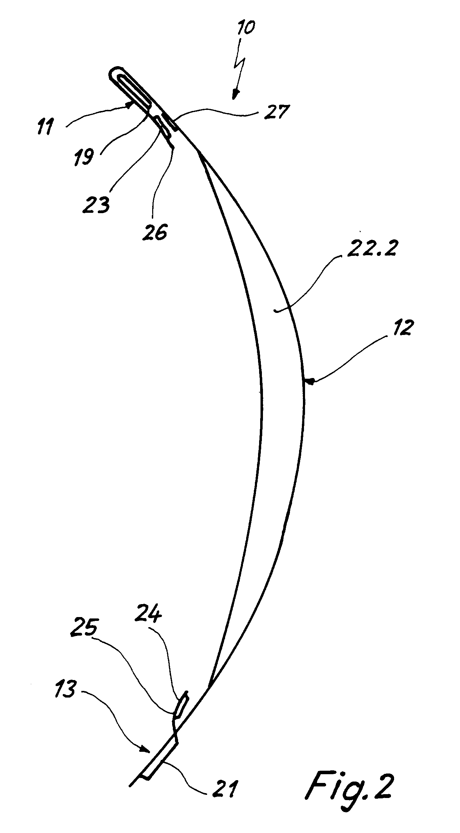

Fig. 2 zeigt die Windelhose 10 im Schnitt in ihrer leicht gebogenen Form. Am oberen Ende der Windelhose 10 ist der hintere Bundteil 11 mit dem Gummiband 19 zu sehen und darunter das Stoffband 26 mit dem an ihm befestigten Klettband 23. Dem Klettband 23 gegenüber ist eine Flauschfläche 27 an der Windel befestigt. An den hinteren Bundteil 11 schließt sich das Mittelteil 12 mit dem Auslaufschutz 22.2 an. Im unteren Bereich der Figur ist der vordere Bundteil 13 mit der Auflagefläche 21 zu sehen. Das Stoffband 25 ist zum Mittelteil 12 hin an den vorderen Bundteil 13 anschließend am Bund befestigt und trägt ein Klettband 24, das vom Stoffband 25 verdeckt wird.

## Patentansprüche

1. Windelhose (10) zum Einlegen einer Windel mit einem hinteren Bundteil (11), einem Mittelteil (12) und einem vorderen Bundteil (13), bei der der hintere Bundteil (11) Verschlußlaschen (14.1, 14.2) und einen Stofftunnel (17) aufweist, in der ein Bundgummiband (19) geführt ist, das auf beiden Längsenden des Stofftunnels (17) an dem Stofftunnel fixiert ist und bei der der vordere Bundteil (13) eine Auflagefläche (21) zur Befestigung der Verschlußlaschen (14.1, 14.2) hat, dadurch gekennzeichnet, daß zwischen dem Stofftunnel (17) und mindestens einer Verschlußlasche (14.1, 14.2) eine Pufferzone (18.1, 18.2) angeordnet ist, in der ein Puffergummiband (20.1, 20.2) vorgesehen ist.

2. Windelhose (10) nach Anspruch 1, dadurch gekennzeichnet, daß die Verschlußlaschen (14.1, 14.2) Klettbänder und die Auflagefläche (21) eine Flauschfläche aufweisen.

3. Windelhose (10) nach Anspruch 1 oder 2, dadurch gekennzeichnet, daß das Bundgummiband (19) im ungedehnten Zustand kürzer ist als der Stofftunnel (17).

4. Windelhose (10) nach einem der Ansprüche 1 bis 3, dadurch gekennzeichnet, daß das Puffergummiband (20.1, 20.2) im ungedehnten Zustand kürzer ist als die Pufferzone (18.1, 18.2).

5. Windelhose (10) nach einem der Ansprüche 1 bis 4, dadurch gekennzeichnet, daß Bundgummiband (19) und Puffergummiband (20.1, 20.2) aus einem durchgehenden Gummiband bestehen.

6. Windelhose (10) nach einem der Ansprüche 1 bis 5, dadurch gekennzeichnet, daß das hintere und/oder das vordere Bundteil (11, 13) ein Klettband (23, 24) aufweist, an das über Flauschflächen eine Windel befestigbar ist.

7. Windelhose (10) nach einem der Ansprüche 1 bis 6, dadurch gekennzeichnet, daß auf beiden Seiten des Mittelteils (12) ein außen gerafftes Ansatzstück (22.1, 22.2) als Auslaufschutz angeordnet ist.

8. Windelhose (10) nach einem der Ansprüche 1 bis 7, dadurch gekennzeichnet, daß die Windelhose im wesentlichen aus Baumwolle gefertigt ist.

9. Windelhose (10) nach einem der Ansprüche 1 bis 8, dadurch gekennzeichnet, daµ die Windelhose aus einem wasserundurchlässigen aber atmungsaktiven Material gefertigt ist.
